Europäisches Patentamt

European Patent Office

Office européen des brevets

(11) Publication number: **0 157 604 B1**

# EUROPEAN PATENT SPECIFICATION

(45) Date of publication of patent specification: **30.12.92** (51) Int. Cl.⁵: **C12N 15/57**

(21) Application number: **85302188.9**

(22) Date of filing: **28.03.85**

(54) **Porcine pancreatic elastase.**

(30) Priority: **29.03.84 JP 61744/84**

(43) Date of publication of application:
**09.10.85 Bulletin 85/41**

(45) Publication of the grant of the patent:
**30.12.92 Bulletin 92/53**

(84) Designated Contracting States:
**AT BE CH DE FR GB IT LI NL SE**

(56) References cited:
**EP-A- 0 073 029**
**EP-A- 0 081 831**
**FR-A- 2 428 075**

**BIOCHEMISTRY, vol. 21, 1982, pages 1453-1463, American Chemical Society; R.J. MacDONALD et al.: ""Primary structure of two distinct rat pancreatic prepoelastases determined by sequence analysis of the complete cloned messenger ribonucleic acid sequences"**

**NATURE, vol. 225, 28th Februrary 1970, pages 811-816; D.M. SHOTTON et al.: "Three-dimensional structure of tosyl-elastase"**

(73) Proprietor: **SANKYO COMPANY LIMITED No. 1-6, 3-chome Nihonbashi Honcho Chuo-ku Tokyo(JP)**

(72) Inventor: **Takiguchi, Yo c/o Bio-science Research Lab Sankyo Co., Ltd 2-58, 1-chome, Hiromachi Shinagawa-ku Tokyo(JP)**
Inventor: **Tani, Tokio c/o Bio-science Research Lab Sankyo Co., Ltd 2-58, 1-chome, Hiromachi Shinagawa-ku Tokyo(JP)**
Inventor: **Kawashima, Ichiro c/o Bio-science Research Lab Sankyo Co., Ltd 2-58, 1-chome, Hiromachi Shinagawa-ku Tokyo(JP)**
Inventor: **Ohmine, Toshinori c/o Bio-science Research Lab Sankyo Co., Ltd 2-58, 1-chome, Hiromachi Shinagawa-ku Tokyo(JP)**
Inventor: **Furukawa, Hidehiko c/o Bio-science Research Lab Sankyo Co., Ltd 2-58, 1-chome, Hiromachi Shinagawa-ku Tokyo(JP)**

CHEMICAL ABSTRACTS, vol. 105, no. 3, 21st July 1986, page 225, abstract no. 19848r, Columbus, Ohio, US; & JP-A-60 186 284 (KIRIN BREWERY CO., LTD) 21-09-1985

BIOCHIMICA ET BIOPHYSICA ACTA, vol. 623, 1980, pages 208-212, Elsevier/North-Holland Biomedical Press; C. LARGMAN et al.: "Human pacreatic proelastase 2 - Sequence of the activation peptide"

Inventor: **Momota, Kenji c/o Bio-science Research Lab**
**Sankyo Co., Ltd 2-58, 1-chome, Hiromachi Shinagawa-ku Tokyo(JP)**

(74) Representative: **Gibson, Christian John Robert et al**
**MARKS & CLERK 57/60 Lincoln's Inn Fields London WC2A 3LS(GB)**

## Description

This invention relates to porcine pancreatic elastase.

Elastase is one of the serine proteases, and is an enzyme capable of hydrolyzing the fibrous insoluble protein known as elastin. Elastin is a scleroprotein, forming connective tissues, tendons, aortic integuments and the cervical bundles of higher animals. Elastin can be slightly degraded by pepsin and trypsin.

Balo' et al. observed the degradation of elastin fibres of blood vessel walls in the course of research on arteriosclerosis, and suggested the presence of an enzyme which degrades elastin [Balo'. J. and Banga, I.: Schweiz Z. Pathol. Bacteriol., 12, 350 (1949)]. Subsequently, Banga discovered an enzyme in the pancreas which specifically degraded elastin, isolated it as crystals, and named it "elastase" [Banga, I.: Acta Physiol. Acad. Sci. Hung., 3, 317 (1952)]. The structure of porcine pancreatic elastase has been elucidated [see, for example, Shotton, D.M. and Watson, H.C.: Nature, 225, 811 (1970].

It has been confirmed that elastases exist in the pancreas of most animals, including human beings, monkeys, cats, rabbits. etc., with the level being about 3.1 mg/g-pancreas in human beings, about 2.2 mg/g in bovids and about 10.2 mg/g in rats. A correlation between the elastase activity and the age of human beings has been observed: elastase activity was markedly low in the pancreas and plasma of males of 40 years or more and females of 60 years or more [Loeven, W.A., and Baldwin, Maureen M.: Gerontologia, 17, 170 (1971)].

In the case of patients with arteriosclerosis, the elastase activity in the pancreas was found to be markedly low, compared with normally healthy people [Balo', J., and Banga, I.: Nature, 178, 310 (1956)]. It has been shown by subsequent studies that elastase not only enzymatically degrades elastin but also promotes elastin biosynthesis.

Studies on the pharmacological action of elastase have been made using rats or rabbits, to reveal the following effects:

1) inhibition of deposition of lipids and calcium on arterial walls:

2) elimination of cholesterol and calcium from arterial walls:

3) selective decomposition of denatured elastin:

4) promotion of growth of fresh elastin fibres in arterial walls;

5) action of lowering serum lipids: and

6) action of improving lipoprotein metabolism.

In clinical studies conducted on the basis of the pharmacological studies, the following effects have been observed:

1) restoration of elasticity and expandability of arterial walls;

2) improvement of serum lipid abnormality; and

3) improvement of lipoprotein metabolism.

In the above studies, the elastase had been extracted and purified from porcine pancreas. However, the starting material for such an extraction is not necessarily abundant and is relatively expensive. Furthermore it is frequently difficult to procure sufficient amounts of porcine pancreas.

It is an object of this invention to make porcine pancreatic elastase more readily available, with the possibility of obtaining the elastase in substantially pure form. It is a further object of this invention to eliminate the continuing dependency on porcine pancreas for adequate supplies of porcine pancreatic elastase. It is a yet further object to open up the possibility of producing elastase-like compounds.

This invention applies the techniques of genetic engineering to the production of porcine pancreatic elastase. By the use of recombinant DNA technology in accordance with the present invention, it is now possible to provide a base sequence coding for porcine pancreatic elastase or a functionally similar compound. Correspondingly, the elastase and functionally similar compounds now become readily available for the first time.

Through the use of genetically engineered sources, it is now possible in accordance with the present invention to provide a DNA which codes for a compound capable of functioning as a porcine pancreatic elastase. Such compounds (which include pro-forms and pre-pro-forms as well as compounds arising from silent mutations and other compounds functionally effective as elastases) are included within the expression "genetically engineered porcine pancreatic elastase" and within analogous expressions.

Thus, for example, compounds which function as a porcine pancreatic elastase but which are not identical in structure to naturally occuring porcine pancreatic elastase are included within the expression "porcine pancreatic elastase from a genetically engineered source". Compounds with one or more changed amino acids, and compounds with one or more extra amino acids, are included. In particular, proelastases and preproelastases, when expressed by a recombinant DNA sequence, as well as elastases obtained by activation of such precursor compounds are included within "porcine pancreatic elastase from a genetically engineered source".

More particularly, the DNA of this invention preferably includes a base sequence of the following formula (I):

```
(5') GTA-GTT-GGA-GGG-ACC-GAG-GCT-CAG-AGG-AAT-

     TCT-TGG-CCA-TCT-CAG-ATT-TCC-CTC-CAG-TAC-

     CGG-TCT-GGA-AGT-TCG-TGG-GCT-CAC-ACC-TGT-

     GGA-GGG-ACC-CTC-ATC-AGG-CAG-AAC-TGG-GTG-

     ATG-ACA-GCC-GCT-CAC-TGC-GTG-GAC-AGA-GAG-

     TTG-ACC-TTC-CGT-GTG-GTG-GTT-GGA-GAG-CAC-

     AAC-CTG-AAC-CAG-AAC-GAT-GGC-ACC-GAG-CAG-

     TAC-GTG-GGG-GTG-CAG-AAG-ATC-GTG-GTG-CAT-

     CCC-TAC-TGG-AAC-ACC-GAC-GAC-GTG-GCT-GCA-

     GGC-TAT-GAC-ATC-GCC-CTG-CTG-CGC-CTG-GCC-

     CAG-AGT-GTA-ACC-CTC-AAC-AGC-TAC-GTC-CAG-

     CTG-GGT-GTT-CTG-CCA-AGG-GCG-GGG-ACC-ATC-

     CTG-GCT-AAC-AAC-AGT-CCC-TGC-TAC-ATC-ACA-

     GGC-TGG-GGC-CTG-ACC-AGG-ACC-AAT-GGG-CAG-

     CTG-GCC-CAG-ACC-CTG-CAG-CAG-GCT-TAC-CTG-

     CCC-ACC-GTG-GAC-TAC-GCC-ATC-TGC-TCC-AGC-

     TCC-TCG-TAC-TGG-GGC-TCC-ACC-GTG-AAG-AAC-


     AGC-ATG-GTG-TGC-GCC-GGA-GGG-GAC-GGA-GTT-

     CGC-TCT-GGA-TGT-CAG-GGT-GAC-TCT-GGG-GGC-

     CCC-CTT-CAT-TGC-TTG-GTG-AAT-GGT-CAG-TAT-

     GCT-GTC-CAC-GGT-GTA-ACC-AGC-TTC-GTG-TCC-

     CGC-CTG-GGC-TGT-AAT-GTC-ACC-AGG-AAG-CCC-

     ACA-GTC-TTC-ACC-AGG-GTC-TCT-GCT-TAC-ATC-

     TCT-TGG-ATA-AAT-AAC-GTC-ATT-GCC-AGC-AAC  (3')
```

Such a base sequence can result in expression of a protein which includes an amino acid sequence of the

4

following formula (II):

```
Val-Val-Gly-Gly-Thr-Glu-Ala-Gln-Arg-Asn-
Ser-Trp-Pro-Ser-Gln-Ile-Ser-Leu-Gln-Tyr-
Arg-Ser-Gly-Ser-Ser-Trp-Ala-His-Thr-Cys-
Gly-Gly-Thr-Leu-Ile-Arg-Gln-Asn-Trp-Val-
Met-Thr-Ala-Ala-His-Cys-Val-Asp-Arg-Glu-
Leu-Thr-Phe-Arg-Val-Val-Val-Gly-Glu-His-
Asn-Leu-Asn-Gln-Asn-Asp-Gly-Thr-Glu-Gln-
Tyr-Val-Gly-Val-Gln-Lys-Ile-Val-Val-His-
Pro-Tyr-Trp-Asn-Thr-Asp-Asp-Val-Ala-Ala-
Gly-Tyr-Asp-Ile-Ala-Leu-Leu-Arg-Leu-Ala-
Gln-Ser-Val-Thr-Leu-Asn-Ser-Tyr-Val-Gln-
Leu-Gly-Val-Leu-Pro-Arg-Ala-Gly-Thr-Ile-
Leu-Ala-Asn-Asn-Ser-Pro-Cys-Tyr-Ile-Thr-
Gly-Trp-Gly-Leu-Thr-Arg-Thr-Asn-Gly-Gln-


Leu-Ala-Gln-Thr-Leu-Gln-Gln-Ala-Tyr-Leu-
Pro-Thr-Val-Asp-Tyr-Ala-Ile-Cys-Ser-Ser-
Ser-Ser-Tyr-Trp-Gly-Ser-Thr-Val-Lys-Asn-
Ser-Met-Val-Cys-Ala-Gly-Gly-Asp-Gly-Val-
Arg-Ser-Gly-Cys-Gln-Gly-Asp-Ser-Gly-Gly-
Pro-Leu-His-Cys-Leu-Val-Asn-Gly-Gln-Tyr-
Ala-Val-His-Gly-Val-Thr-Ser-Phe-Val-Ser-
Arg-Leu-Gly-Cys-Asn-Val-Thr-Arg-Lys-Pro-
Thr-Val-Phe-Thr-Arg-Val-Ser-Ala-Tyr-Ile-
Ser-Trp-Ile-Asn-Asn-Val-Ile-Ala-Ser-Asn
```

It is to be recognized that such an amino acid sequence can arise from different DNA base sequences to the one given above, and such modified DNA sequences are also part of this invention.

In particular, the genetically engineered elastase of this invention can be of the formula (III):

5

```
Y-Val-Val-Gly-Gly-Thr-Glu-Ala-Gln-Arg-Asn-

  Ser-Trp-Pro-Ser-Gln-Ile-Ser-Leu-Gln-Tyr-

  Arg-Ser-Gly-Ser-Ser-Trp-Ala-His-Thr-Cys-

  Gly-Gly-Thr-Leu-Ile-Arg-Gln-Asn-Trp-Val-

  Met-Thr-Ala-Ala-His-Cys-Val-Asp-Arg-Glu-

  Leu-Thr-Phe-Arg-Val-Val-Val-Gly-Glu-His-

  Asn-Leu-Asn-Gln-Asn-Asp-Gly-Thr-Glu-Gln-


  Tyr-Val-Gly-Val-Gln-Lys-Ile-Val-Val-His-

  Pro-Tyr-Trp-Asn-Thr-Asp-Asp-Val-Ala-Ala-

  Gly-Tyr-Asp-Ile-Ala-Leu-Leu-Arg-Leu-Ala-

  Gln-Ser-Val-Thr-Leu-Asn-Ser-Tyr-Val-Gln-

  Leu-Gly-Val-Leu-Pro-Arg-Ala-Gly-Thr-Ile-

  Leu-Ala-Asn-Asn-Ser-Pro-Cys-Tyr-Ile-Thr-

  Gly-Trp-Gly-Leu-Thr-Arg-Thr-Asn-Gly-Gln-

  Leu-Ala-Gln-Thr-Leu-Gln-Gln-Ala-Tyr-Leu-

  Pro-Thr-Val-Asp-Tyr-Ala-Ile-Cys-Ser-Ser-

  Ser-Ser-Tyr-Trp-Gly-Ser-Thr-Val-Lys-Asn-

  Ser-Met-Val-Cys-Ala-Gly-Gly-Asp-Gly-Val-

  Arg-Ser-Gly-Cys-Gln-Gly-Asp-Ser-Gly-Gly-

  Pro-Leu-His-Cys-Leu-Val-Asn-Gly-Gln-Tyr-

  Ala-Val-His-Gly-Val-Thr-Ser-Phe-Val-Ser-

  Arg-Leu-Gly-Cys-Asn-Val-Thr-Arg-Lys-Pro-

  Thr-Val-Phe-Thr-Arg-Val-Ser-Ala-Tyr-Ile-

  Ser-Trp-Ile-Asn-Asn-Val-Ile-Ala-Ser-Asn
```

where the group Y is a hydrogen atom,

                                          Met,  Thr-Gln-Asp-

Phe-Pro-Glu-Thr-Asn-Ala-Arg   or   Met-Leu-Arg-Leu-Leu-Val-

Val-Ala-Ser-Leu-Val-Leu-Tyr-Gly-His-Ser-Thr-Gln-Asp-Phe-Pro-Glu-Thr-Asn-Ala-Arg.

Such compounds can be obtained using a DNA having a base sequence represented by the following formula (IV), or a base sequence of the same effect:

(5') Z-GTA-GTT-GGA-GGG-ACC-GAG-GCT-CAG-AGG-AAT-

TCT-TGG-CCA-TCT-CAG-ATT-TCC-CTC-CAG-TAC-

CGG-TCT-GGA-AGT-TCG-TGG-GCT-CAC-ACC-TGT-

GGA-GGG-ACC-CTC-ATC-AGG-CAG-AAC-TGG-GTG-

ATG-ACA-GCC-GCT-CAC-TGC-GTG-GAC-AGA-GAG-

TTG-ACC-TTC-CGT-GTG-GTG-GTT-GGA-GAG-CAC-

AAC-CTG-AAC-CAG-AAC-GAT-GGC-ACC-GAG-CAG-

TAC-GTG-GGG-GTG-CAG-AAG-ATC-GTG-GTG-CAT-

CCC-TAC-TGG-AAC-ACC-GAC-GAC-GTG-GCT-GCA-

GGC-TAT-GAC-ATC-GCC-CTG-CTG-CGC-CTG-GCC-

CAG-AGT-GTA-ACC-CTC-AAC-AGC-TAC-GTC-CAG-

CTG-GGT-GTT-CTG-CCA-AGG-GCG-GGG-ACC-ATC-

CTG-GCT-AAC-AAC-AGT-CCC-TGC-TAC-ATC-ACA-

GGC-TGG-GGC-CTG-ACC-AGG-ACC-AAT-GGG-CAG-

CTG-GCC-CAG-ACC-CTG-CAG-CAG-GCT-TAC-CTG-

CCC-ACC-GTG-GAC-TAC-GCC-ATC-TGC-TCC-AGC-

TCC-TCG-TAC-TGG-GGC-TCC-ACC-GTG-AAG-AAC-

AGC-ATG-GTG-TGC-GCC-GGA-GGG-GAC-GGA-GTT-

CGC-TCT-GGA-TGT-CAG-GGT-GAC-TCT-GGG-GGC-

CCC-CTT-CAT-TGC-TTG-GTG-AAT-GGT-CAG-TAT-

GCT-GTC-CAC-GGT-GTA-ACC-AGC-TTC-GTG-TCC-

CGC-CTG-GGC-TGT-AAT-GTC-ACC-AGG-AAG-CCC-

ACA-GTC-TTC-ACC-AGG-GTC-TCT-GCT-TAC-ATC-

TCT-TGG-ATA-AAT-AAC-GTC-ATT-GCC-AGC-AAC-X (3')

wherein Z represents

ATG, ACC-CAG-GAC-TTT-CCA-GAA-ACC-

AAC-GCC-CGG or ATG-CTG-CGC-TTG-CTG-GTG-GTG-GCC-TCC-CTG-

8

GTC-CTT-TAT-GGA-CAC-AGC-ACC-CAG-GAC-TTT-CCA-GAA-ACC-AAC-GC

C- CGG,

and X represents a stop codon, that is TAA, TGA or TAG.

When Z represents ACC-CAG-GAC-TTT-CCA-GAA-ACC-AAC-GCC-CGG, then the resultant compound will include the amino acid sequence for the activation peptide of elastase, Thr-Gln-Asp-Phe-Pro-Glu-Thr-Asn-Ala-Arg. When Z represents

ATG-CTG-CGC-TTG-CTG-GTG-GTG-GCC-TCC-CTG-GTC-

CTT-TAT-GGA-CAC-AGC-ACC-CAG-GAC-TTT-CCA-GAA-ACC-AAC-GCC-CG

G,

then the resultant compound will include the amino acid sequence for the signal peptide and the activation peptide of elastase,

Met-Leu-Arg-Leu-Leu-Val-Val-Ala-

Ser-Leu-Val-Leu-Tyr-Gly-His-Ser-Thr-Gln-Asp-Phe-Pro-Glu-Thr-Asn-Ala-Arg.

Such peptides can be removed by hydrolysis. When Z represents ATG, then Y represents Met. It will readily be appreciated that the group Y can take other forms, and an example is given below.

The DNA of this invention including the base sequence represented by the formula (I) can be produced for example in accordance with the steps (a) to (g):

(a) obtaining mRNA from porcine pancreas;
(b) synthesizing single-stranded DNA complementary to the mRNA:
(c) producing double-stranded DNA from the single stranded cDNA;
(d) inserting the double-stranded DNA into a vector to give a recombinant DNA;
(e) transforming a host with the recombinant DNA;
(f) cloning the transformed host; and
(g) isolating the desired DNA from the cloned host.

From the DNA, the corresponding amino acid sequence can then be obtained for example by the steps of:

(1) inserting the DNA in to an expression vector;
(2) transforming a host organism:
(3) culturing the transformed host under conditions resulting in expression of the DNA sequence; and
(4) isolating a compound including the amino acid sequence.

The steps (a) to (g) for producing the DNA sequence typically involve:

(a) obtaining mRNA from porcine pancreas including the mRNA coding for porcine pancreatic elastase;
(b) synthesizing single-stranded cDNA using the mRNA and a reverse transcriptase;
(c) synthesizing double-stranded DNA on the basis of the single-stranded cDNA,
(d) inserting the double-stranded DNA into a vector to give a recombinant plasmid;
(e) transforming a host such as Escherichia coli, Bacillus subtilis, a yeast or an animal cell, by introducing the recombinant plasmid;
(f) cultivating the transformed host to obtain cloned cells containing a plasmid with DNA coding for porcine pancreatic elastase; and
(g) excising the desired cloned DNA from the identified plasmid.

The mRNA should desirably be extracted from the porcine pancreas as quickly as possible after excision of the pancreas from a freshly killed pig. If this approach is impossible, the pancreas should preferably be frozen with liquid nitrogen and stored at - 80°C until the mRNA extraction.

In extracting the RNA from the pancreas, various methods can be used including the guanidine-thiocyanate-hot-phenol method, the guanidine-thiocyanate-cesium chloride method or the guanidine-thiocyanate-guanidine hydrochloride method. The last mentioned method is superior in the following respects: (1) the procedure is simpler; (2) recovery from extraction is high; (3) extraction from a relatively large number of pancreas is possible; (4) no DNA is entrained; and (5) degradation of RNA is low.

Most of the mRNA existing in the cytoplasm of eukaryotic cells has a poly(A) sequence at the 3'-end. Utilizing this structural feature, the extracted mRNA can be purified by adsorbing it on to an oligo(dT) cellulose column and subsequently eluting it.

Using the mRNA as the template, a complementary DNA can be synthesized through the use of a reverse transcriptase, and a double-stranded DNA prepared. As the synthetic method, there may be employed the $S_1$ nuclease method [Efstratiadis, A. et al. : Cell, 7, 279 (1976)], the Land method (Land, H. et al.: Nucleic Acids Res., 9, 2251 (1981)], the Okayama-Berg method [Okayama, H. and Berg P.: Mol. Cell. Biol., 2, 161 (1982)], the O. Joon Yoo method [O. Joon Yoo et al.: Proc. Natl. Acad. Sci. USA, 79, 1049 (1982)], etc. For the objects of the present invention, the O. Joon Yoo method was found to be preferable.

The double-stranded cDNA can then be inserted into a suitable vector. The vector typically takes the form of a plasmid especially a plasmid with a unique restriction endonuclease site, more especially a restriction site within a phenotypic sequence. Various insertion techniques can be employed, but for preference homopolymer tailing is employed. A short homopolymer chain of one DNA (dA, dT, dC or dG) is applied to each 3'-end of the cDNA, and the plasmid vector is cleaved open and similarly tailed using a complementary DNA (dA for dT, etc).

For example, homopolymer tailing is applied to the cDNA, and the tailed product is inserted into the correspondingly cleaved and tailed plasmid pBR322.

The site cleaved with the restriction endonuclease Pst I in pBR322 is preferred. The homopolymer pairs for tailing are preferably dG and dC in view of the better stability.

Next, the recombinant plasmid can be introduced in to a suitable host, suitably E. coli, for example, the known X1776 strain. The transformed organism can then be selected on the basis of phenotype modification, using for instance tetracycline resistance or ampicillin resistance as the marker. In the most preferred aspect of the present invention, the cDNA is inserted into plasmid pBR322 at the ampicillin resistant gene site, and so colonies of E. coli sensitive to ampicillin and resistant to tetracycline can be selected.

In order to select from the recombinant organisms obtained the clones containing cDNA coding for an elastase, it is possible to employ the colony hybridization method. In this method, an oligonucleotide having a base sequence corresponding to the desired amino acid sequence is chemically synthesized and labelled with $^{32}$P, and the oligonucleotide is used as a probe [Grunstein M. and Hogness D.S.: Proc. Natl. Acad. Sci. USA, 72, 3961 (1975)]. However, in the present invention, the direct sequencing method is preferably employed for the selection of clones.

Determination of the base sequence contained in the selected clone may be performed according to the method of dideoxynucleotide termination sequencing with the use of phage M13 [Messing, J. et al.: Nucleic Acids Res., 9, 309 (1981)] and the Maxam-Gilbert method [Maxam, A.M. and Gilbert, W.: Proc. Natl. Acad. Sci. USA, 74, 560 (1977)]. In the present invention, the Maxam-Gilbert method is preferably employed for the base sequencing and a clone having a complete DNA coding for the elastase of porcine pancreas is identified.

As the next step, the elastase cDNA or a longer sequence containing the elastase cDNA can be excised from the cloned DNA, coupled with an appropriate expression vector and introduced into an appropriate host to effect expression.

The promoter for the expression vector may be for instance the tryptophan (trp) promoter, lactose (lac) promoter. tryptophan-lactose (tac) promoter, lipoprotein (lpp) promoter, lambda ($\lambda$) $P_L$ promoter derived from bacteriophage or the protein chain elongating factor Tu (tuf B) promoter.

The host can be bacteria such as E. coli and B. subtilis, though yeast or animal cells are alternatives.

In the present invention, E. coli (LE392, JM103, etc.) is preferably employed as the host for the expression of protein.

For transformation of the host by the cDNA in accordance with the preferred method, there may be employed the rubidium chloride method [Kushner, S.R.: Genetic Engineering (eds. Boyer, H.W. and Niscosia, S.), p. 17, Elsevier (1978)], the calcium chloride method [Dagert, M. and, Ehrlich, S.D.: Gene, 6, 23 (1979)], the low pH method [Manual of Genetic Manipulation, edited by Yasutaka Takagi; p. 49 Kodansha Scientific (1982)], etc. In the present invention, the calcium chloride method is preferably employed.

The recombinant organism thus obtained is cultivated in a known medium to produce and accumulate the elastase or a substance having the same effect, followed by recovery thereof. Suitable media include those comprising glucose, casamino acid, etc., for example, M9 medium [Miller, J.: Experiments in Molecular Genetics, 431-433 (Cold Spring Harbor Lab., New York (1972)].

The recombinant micro-organism may then be cultivated generally at 15 to 43°C for 3 to 24 hours, under aeration and stirring, if necessary. After cultivation, the organisms can be harvested in a conventional manner, suspended in a buffer to break up the cells, followed by centrifugation to obtain the supernatant. For breaking the cells, there may be employed, for example, a sonification treatment, lysozyme treatment or freeze-thaw treatment. Isolation of elastase from the said supernatant can be performed according to the conventional methods for purification of proteins.

The typical elastase produced by the present invention exhibits the same biological activity as the elastase purified by extraction from porcine pancreas, and can be used for the same purposes and in the same manner.

The present invention is illustrated by the following non-limiting examples taken in conjunction with the accompanying drawings, in which:

Figure 1 shows the primary structure, together with the encoded amino acid sequence, for the plasmid pPE603 produced in Example 1;

Figure 2 shows a restriction endonuclease map of the plasmid pPE603 produced in Example 1; and

Figure 3 shows part of the primary structure and encoded amino acid sequence for plasmid pPE777 obtained in Example 1 and used in Example 2;

Figure 4 shows part of the primary structure and encoded amino acid sequence for known plasmid pUC8 employed in Example 2;

Figure 5 shows the construction of a plasmid pPEX777 produced in Example 2; and

Figure 6 shows part of the primary structure and encoded amino acid sequence for the plasmid pPEX777 produced in Example 2.

Example 1

(1) isolation of mRNA from porcine pancreas

Porcine pancreas weighing 8 to 9 g was homogenized and denatured in an homogenizer (a Polytron homogenizer from Kinematica GmbH Germany) in an adequate amount of guanidinethiocyanate solution (4 M guanidinethiocyanate; 1% detergent, Sarcosyl from Sigma Chemical Co., USA; 20 mM ethylenediaminetetraacetic acid, EDTA; 25 mM sodium citrate, pH 7.0; 100 mM 2-mercaptoethanol; and 0.1% defoaming agent, Antifoam A from Sigma Chemical Co., USA). The homogenized mixture was then centrifuged and the supernatant retained.

1 volume of the supernatant was mixed with 0.025 volumes of 1 M acetic acid and 0.75 volumes of ethanol, and the mixture cooled at -20°C for several hours, followed by centrifugation, to obtain a precipitate. The precipitate was then suspended in a guanidine hydrochloride solution (7.5 M quanidine hydrochloride; 25 mM sodium citrate, pH 7.0; and 5 mM dithiothreitol, DTT). 1 volume of the suspension was mixed with 0.025 volumes of 1 M acetic acid and 0.5 volumes of ethanol, and the resultant mix cooled to -20°C for several hours, followed by centrifugation. The centrifuged precipitate was suspended again in more of the guanidine hydrochloride solution, mixed as before with acetic acid and ethanol, cooled to -20°C and centrifuged, followed by collection of the precipitate. Next, the precipitate was washed several times with ethanol to remove excess guanidine hydrochloride and dissolved in distilled water, followed by precipitation of the RNA with ethanol. The precipitate was collected using centrifugation to give 53.9 mg of RNA.

The RNA thus obtained was adsorbed onto an oligo(dT) cellulose column in a saline buffer solution (0.5 M NaCl; 20 mM Tris-HCl, pH 7.5; 1 mM EDTA; and 0.1% sodium dodecylsulfate, SDS), and mRNA containing poly(A) was eluted with a buffer solution (10 mM Tris-HCl, pH 7.5, 1 mM EDTA and 0.05% SDS) to give 540 $\mu$g of mRNA.

(2) synthesis of single-stranded cDNA

In order to synthesize a single-stranded cDNA with an oligo(dT) primer, the mRNA was incubated at 42°C for one hour in 50$\mu$l of a suitable reaction mixture (10 $\mu$g of mRNA; 10 $\mu$g of oligo(dT)$_{16}$; 50 mM Tris-HCl, pH 8.3; 50 mM of KCl; 10 mM of MgCl$_2$; 10 mM of DTT; 1 mM of each of dCTP, dGTP and dTTP; 0.6 mM of dATP; 50 $\mu$Ci of $\alpha$-$^{32}$P-dATP; and 50 units of reverse transcriptase). After the incubation, nucleic acids with low molecular weights were removed from the reaction product using column chromatography with Sephadex G-200 (trade mark, Pharmacia Co., Sweden), followed by incubation of the remaining reaction product in a solution of 0.3 M NaOH and 2 mM EDTA at 23°C for 16 hours, in order to degrade and thereby remove RNA from the desired single-stranded cDNA.

(3) synthesis of double-stranded cDNA

A chain of 20 or more residues of deoxyadenosine was attached to the 3'-end of the single-stranded cDNA by incubation at 30°C for 20 minutes in 50 $\mu$l of a suitable reaction mixture (0.1 M potassium cacodylate of formula $C_2H_6AsKO_2$ and pH 7.0; 100 $\mu$g/ml bovine serum albumin, BSA; 0.1 mM DTT; 1 mM $CaCl_2$; 0.6 mM dATP; and 50 units of terminal transferase).

The cDNA with deoxyadenosine tail was collected by ethanol precipitation and then incubated in 50 $\mu$l of a reaction mixture (10 $\mu$g oligo(dT)$_{16}$; 50 mM Tris-HCl, pH 8.3; 50 mM KCl; 10 mM $MgCl_2$; 10 mM DTT; 1 mM of each of dATP, dCTP, dGTP and dTTP; and 50 units of reverse transcriptase) at 37°C for 10 minutes, then at 42°C for 50 minutes. After the reaction, deproteinization was carried out with phenol, and cDNA was precipitated with ethanol. The precipitate was then collected by centrifugation and allowed to react with DNA polymerase I in 50 $\mu$l of a reaction mixture (50 mM phosphate buffer, pH 7.4; 6.6 mM $MgCl_2$; 1.5 mM DTT; 0.4 mM each of dATP, dCTP, dGTP and dTTP; 7.5 units of DNA polymerase I) at 37°C for 2 hours to synthesize a double-stranded cDNA. By use of a Sephadex G-200 column, any cDNA with low molecular weight was removed, and finally about 600 ng of a double-stranded cDNA was obtained.

(4) addition of a deoxycytidine chain

A deoxycytidine chain of about 20 residues was attached to the 3'-end of both strands of the double-stranded cDNA by incubating the cDNA in 50 $\mu$l of a reaction mixture (0.1 M potassium cacodylate, pH 7.0; 100 $\mu$g/ml of BSA; 0.1 mM DTT; 1 mM $CaCl_2$; 0.2 mM dCTP; and 37.5 units of terminal transferase) at 37°C for one minute.

(5) annealing cDNA with vector and transformation of E. coli

500 ng of the known plasmid pBR322 was cleaved with the restriction endonuclease Pst I and a deoxyguanosine chain of about 20 residues was attached to each 3'-end of the linearized plasmid. 100 ng of the double-stranded cDNA with deoxycytidine tails was mixed with the linearized and tailed plasmid in a saline solution (0.1 M NaCl, 10 mM Tris-HCl (pH 7.5) and 1 mM EDTA solution) and heated at 65°C for 5 minutes. Then, the mixture was gradually cooled to anneal the cDNA with the pBR322 and obtain a recombinant plasmid.

The known strain E. coli X1776 was transformed with the recombinant plasmid thus obtained, according to the method of Komoroff et al. [Proc. Natl. Acad. Sci., USA, 75, 3727 (1978)].

(6) isolation of the plasmid containing elastase cDNA

The strains resulting from the transformation were screened for resistance to tetracycline and sensitivity to ampicillin giving 49 possible strains of interest. From each such strain, a plasmid was isolated according to the method of Birmboim, H.C. and Doly, J. [Nucleic Acids Res., 7, 1513 (1979)], digested with the restriction endonuclease BamH I, and subjected to electrophoresis on agarose gel. The DNA was then transferred from the gel to a nitrocellulose filter and fixed on the filter, in accordance with method of Southern [J. Mol. Biol. , 98, 503 (1975)].

By using 10 $\mu$g porcine pancreatic mRNA isolated in accordance with stage (1) above and 100 $\mu$Ci $\alpha$-$^{32}$P-dATP, single-stranded cDNA labelled with $^{32}$P to a high specific activity was synthesized using the procedure described at stage (2) above. The labelled cDNA was fractionated using agarose gel electrophoresis. The cDNA fraction with a size of around 1000 bases was recovered, this base length corresponding with the mRNA of serine proteases such as elastase, among others.

The labelled cDNA with a length of around 1000 bases was used as a hybridization probe for the DNA material fixed on the nitrocellulose filter. The recombinant plasmids which hybridized with the labelled cDNA of about 1000 base length were detected by autoradiography. It was found that three plasmids hybridized very strongly with the labelled cDNA, and 24 plasmids hybridized moderately therewith.

Next, the primary structure (base sequence) of the cDNA inserted into the plasmids was determined according to the method of Maxam-Gilbert [Proc. Natl. Acad. Sci., USA, 74, 560 (1977)].

From the base sequence determined, the amino acid sequence encoded thereby was deduced. This deduced amino acid sequence was then compared with the known amino acid sequence of porcine pancreatic elastase and the clone giving complete homology in the amino acid sequence was selected. In this way, the plasmid named pPE22 was selected. However, upon further study, it was found that the cDNA inserted in plasmid pPE22 was found to have 480 base pairs, and lacked the region corresponding to the N-terminal end of elastase.

(7) isolation of the plasmid containing complete cDNA

Since plasmid pPE22 did not contain the complete elastase cDNA, and in order to obtain the complete cDNA, the cDNA with deoxycytidine chain at the 3'-end prepared at stage (4) was fractionated using agarose gel electrophoresis. cDNA with 1000 to 1200 base pairs was recovered. The cDNA was annealed as before with the plasmid pBR322. and the known E. coli LE392 was transformed according to the method of Dagert, M. and Ehrlich, S.D. [Gene. 6, 23 (1971)].

1600 transformed strains were obtained. The DNA was extracted from the 1600 strains and fixed onto nitrocellulose filter according to the method of Grunstein, M. and Hogness. D.S. [Proc. Natl. Acad. Sci., USA, 72, 3961 (1975)]. The cDNA fragment contained in plasmid pPE22 was excised with the restriction endonuclease Pst I and labelled with $^{32}$P according to the nick-translation method [Rigby, P.W.J. et al.: J. Mol. Biol.. 113, 237 (1977)]. Using the DNA fragment from pPE22 as a hybridization probe, hybridization with the DNA fixed on the filter was performed according to the method of Alwine et al. 65 clones reactive with the probe were identified by autoradiography.

From these clones, the plasmid DNA was isolated according to the method of Birmboim, H.C. and Doly, J. The isolated DNA was digested with the endonuclease BamH I and subjected to agarose gel electrophoresis. The plasmids named pPE603 and pPE777 containing the longest cDNA were selected.

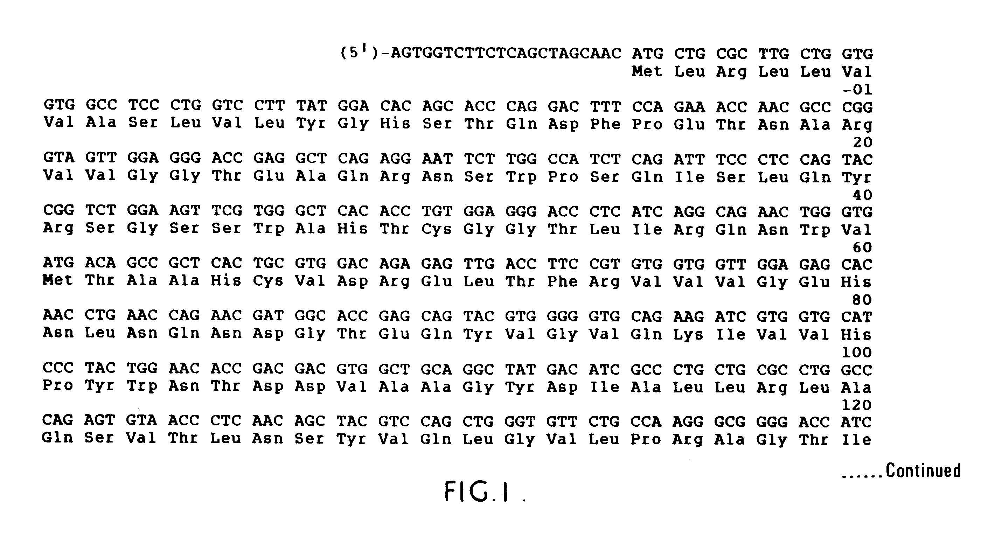

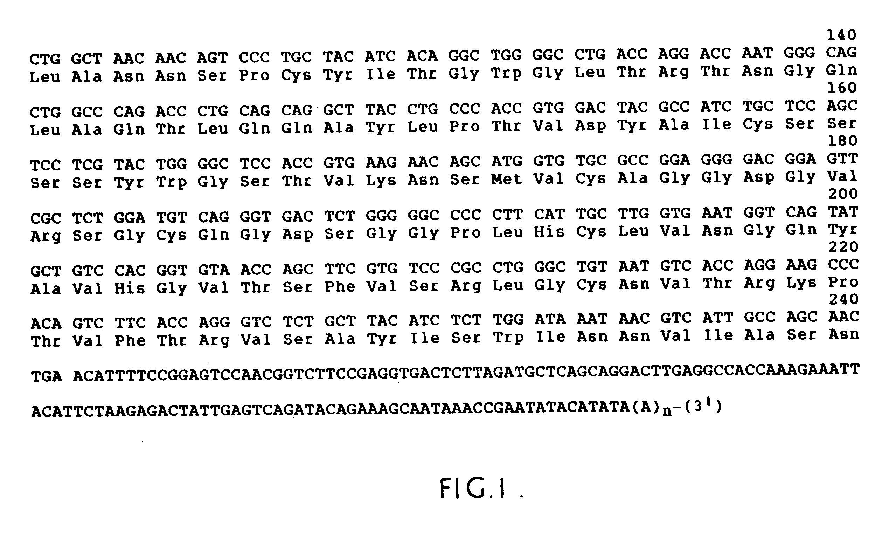

The primary structure for pPE603 as determined by the Maxam-Gilbert method, together with the encoded amino acid sequence, is as shown in Figure 1. It can be seen that plasmid pPE603 has all of the region coding for the signal peptide comprising 16 amino acids, the region coding for the activation peptide comprising 10 amino acids, and the region coding for the mature elastase protein comprising 240 amino acids. Also, plasmid pPE603 contains 5'- and 3'-non-translated regions.

The amino acid sequence is different at two positions to that of the sequence for porcine pancreatic elastase reported by Shotton et al., and novel in that the Asp residues for the amino acids 66 and 178 in the sequence above are different to the Asn residues reported by Shotton et al.

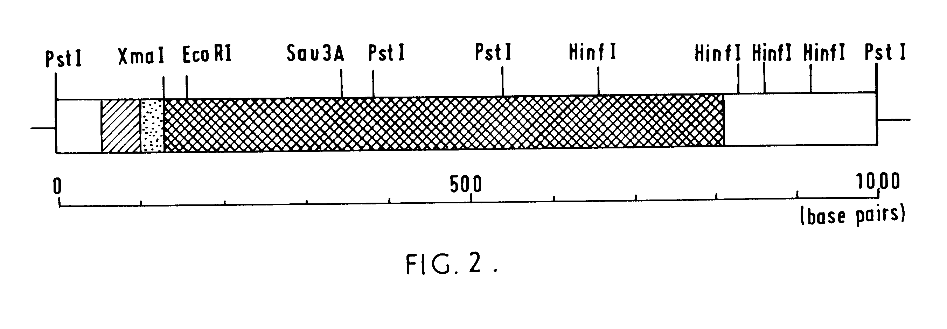

The restriction endonuclease cleavage map of plasmid pPE603 is shown in Figure 2. Reading from the left, the diagonally-marked section represents the region coding for the peptide considered to be the signal peptide, the dot-shaded section represents the region coding for the peptide, considered to be the activation peptide and the cross-shaded section represents the region coding for the peptide considered to be mature elastase protein. Both the signal peptide and the activation peptide were originally found by the present inventors.

The elastase cDNA sequence in pPE777 was found to be inserted in the direction opposite to that in which it was inserted in pPE603.

Example 2

(1) construction of expression vector and transformation of E. coli

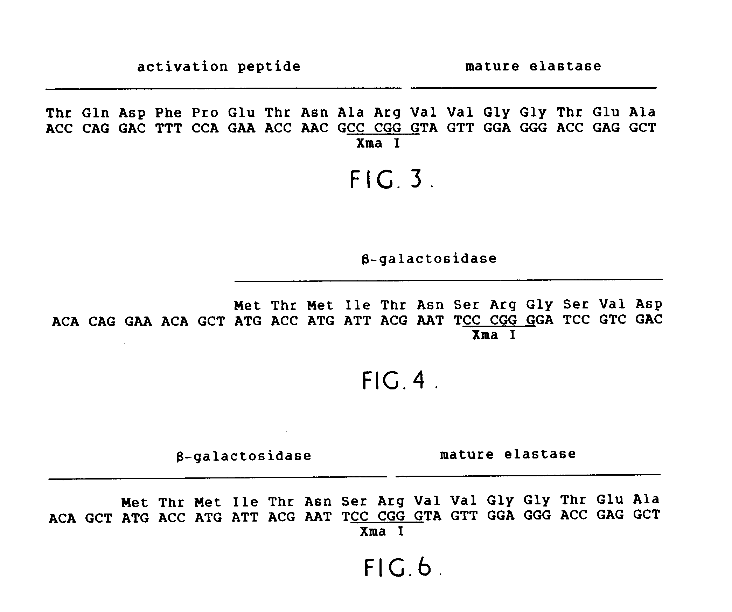

The plasmid pPE777 obtained in Example 1 has a convenient site for the restriction endonuclease Xma I, as shown in Figure 3. The plasmid pPE777 was digested with the restriction endonucleases Xma 1 and Hind III. A DNA fragment of 1.55 kb containing the elastase cDNA and a part of plasmid pBR322 was separated.

Separately, the known plasmid pUC8, which was purchased from Bethesda Research Laboratories, Inc and which includes the DNA sequence shown in Figure 4, was digested with restriction endonucleases Xma 1 and Hind III. The cleavage gave a large fragment of 2.7 kb containing the promoter and operator region of lactose (lac) operon.

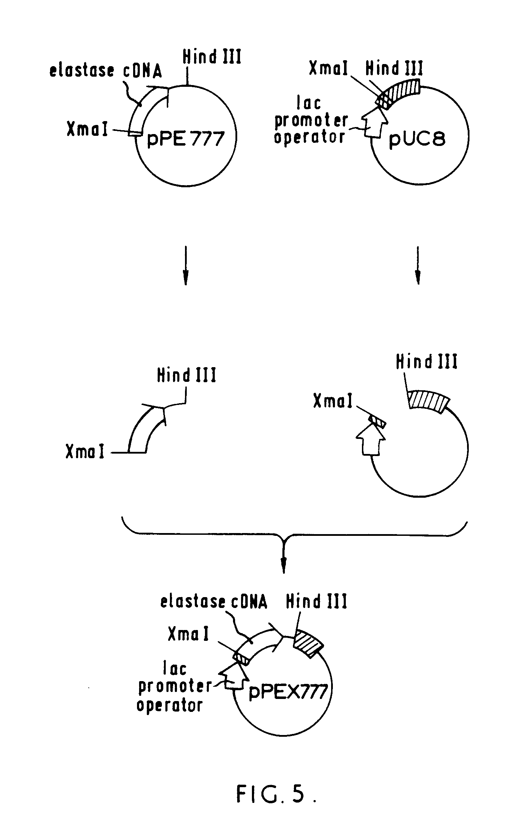

The two kinds of DNA fragment thus obtained were incubated in a 30 μl of a solution containing T4 DNA ligase (66mM Tris-HCl, pH 7.6; 6.6 mM MgCl₂; 10 mM DTT; 1 mM ATP; and 2.5 units of T4 DNA ligase) at 12°C for 16 hours to ligate both DNA fragments at the sites of Xma 1 and Hind III. The expression plasmid thus constructed was named as pPEX777. The construction is illustrated in Figure 5.

E. coli JM103 was transformed by pPEX777 according to the method of Dagert, M. and Ehrlich, S.D. (supra).

(2) expression of the cDNA

The E. coli JM103 containing the elastase expression plasmid pPEX777 was inoculated in an LB-ampicillin medium (10 % bactotrypton, a peptonized protein from Difco, USA; 5 % yeast extract; 10 % NaCl; and 50 μg/ml ampicillin) followed by cultivation overnight at 37°C. 10ml of the culture broth was added to 100ml of a fresh LB-ampicillin medium, followed by shake culture at 37°C up to an optical density OD$_{600}$ of 0.7.

Next, shake culture was continued for an additonal 3 hours, after addition to the culture broth of IPTG (isopropylthio-β-D-galactoside) as an inducer at a final concentration of 2mM. After cultivation, 1 ml of the culture broth was subjected to centrifugation to collect the cultured microorganism, which was then resuspended in 100 μl of a SDS solution (2 % SDS; 5 % 2-mercaptoethanol; 10 % glycerine; and 60 mM Tris-HCl, pH 6.8) After heating at 100°C for 5 minutes, the suspension was subjected to SDS-polyacrylamide gel electrophoresis according to the method of Laemmli [Nature, 227, 680 (1970)].

After the electrophoresis, Western blotting was performed according to the method of Burnette [Anal. Biochem. 112, 195 (1981)], and a protein with a molecular weight of about 26000 reactive with the antibody against porcine elastase was detected with anti-elastase serum and immunoblot kit of BioRad, Inc.

The elastase expressed by pPEX777 was found to be a fused protein in which 8 amino acids derived from β-galactosidase are bound to the N-terminal of the mature elastase, as shown in Figure 6. This finding is easily rationalized on the basis of the Xma I restriction sites given in Figures 3 and 4 for plasmids pPE777 and pUC8.

Thus, a fused protein of porcine elastase and β-galactosidase is formed by cultivating E. coli containing pPEX777 followed by induction with IPTG.

**Claims**

1. An expression vector comprising a cDNA sequence including the following base sequence and coding for porcine pancreatic elastase:

```
(5')   GTA GTT GGA GGG ACC GAG GCT CAG AGG AAT
       TCT TGG CCA TCT CAG ATT TCC CTC CAG TAC
       CGG TCT GGA AGT TCG TGG GCT CAC ACC TGT
       GGA GGG ACC CTC ATC AGG CAG AAC TGG GTG
       ATG ACA GCC GCT CAC TGC GTG GAC AGA GAG
       TTG ACC TTC CGT GTG GTG GTT GGA GAG CAC
       AAC CTG AAC CAG AAC GAT GGC ACC GAG CAG
       TAC GTG GGG GTG CAG AAG ATC GTG GTG CAT
       CCC TAC TGG AAC ACC GAC GAC GTG GCT GCA
       GGC TAT GAC ATC GCC CTG CTG CGC CTG GCC
       CAG AGT GTA ACC CTC AAC AGC TAC GTC CAG
       CTG GGT GTT CTG CCA AGG GCG GGG ACC ATC
       CTG GCT AAC AAC AGT CCC TGC TAC ATC ACA
       GGC TGG GGC CTG ACC AGG ACC AAT GGG CAG
       CTG GCC CAG ACC CTG CAG CAG GCT TAC CTG
       CCC ACC GTG GAC TAC GCC ATC TGC TCC AGC
       TCC TCG TAC TGG GGC TCC ACC GTG AAG AAC
       AGC ATG GTG TGC GCC GGA GGG GAC GGA GTT
       CGC TCT GGA TGT CAG GGT GAC TCT GGG GGC
       CCC CTT CAT TGC TTG GTG AAT GGT CAG TAT
       GCT GTC CAC GGT GTA ACC AGC TTC GTG TCC
       CGC CTG GGC TGT AAT GTC ACC AGG AAG CCC
       ACA GTC TTC ACC AGG GTC TCT GCT TAC ATC
       TCT TGG ATA AAT AAC GTC ATT GCC AGC AAC (3');
```

the expression vector being capable of expressing porcine pancreatic elastase under conditions suitable therefor.

2. An expression vector as claimed in claim 1 wherein the cDNA consists of a DNA sequence of the formula:

```
(5') Z-GTA GTT GGA GGG ACC GAG GCT CAG AGG AAT
     TCT TGG CCA TCT CAG ATT TCC CTC CAG TAC
     CGC TCT GGA AGT TCG TGG GCT CAC ACC TGT
     GGA GGG ACC CTC ATC AGG CAG AAC TGG GTG
     ATG ACA GCC GCT CAC TGC GTG GAC AGA GAG
     TTG ACC TTC CGT GTG GTG GTT GGA GAG CAC
     AAC CTG AAC CAG AAC GAT GGC ACC GAG CAG
     TAC GTG GGG GGG CAG AAG ATC GTG GTG CAT
     CCC TAC TGG AAC ACC GAC GAC GTG GCT GCA
     GGC TAT GAC ATC GCC CTG CTG CGC CTG GCC
     CAG AGT GTA ACC CTC AAC AGC TAC GTC CAG
     CTG GGT GTT CTG CCA AGG GCG GGG ACC ATC
     CTG GCT AAC AAC AGT CCC TGC TAC ATC ACA
     GGC TGG GGC CTG ACC AGG ACC AAT GGG CAG
     CTG GCC CAG ACC CTG CAG CAG GCT TAC CTG
     CCC ACC GTG GAC TAC GCC ATC TGC TCC AGC
     TCC TCG TAC TGG GGC TCC ACC GTG AAG AAC
     AGC ATG GTG TGC GCC GGA GGG GAC GGA GTT
     CGC TCT GGA TGT CAG GGT GAC TCT GGG GGC
     CCC CTT CAT TGC TTG GTG AAT GGT CAG TAT
     GCT GTC CAC GGT GTA ACC AGC TTC GTG TCC
     CGC CTG GGC TGT AAT GTC ACC AGG AAG CCC
     ACA GTC TTC ACC AGG GTC TCT GCT TAC ATC
     TCT TGG ATA AAT AAC GTC ATT GCC AGC AAC-X (3')
```

wherein Z represents

```
                    ACC CAG GAC TTT CCA GAA ACC AAC GCC
  CGG, or ATG CTG CGC TTG CTG GTG GTG GCC TCC CTG GTC CTT
    TAT GGA CAC AGC ACC CAG GAC TTT CCA GAA ACC AAC GCC CGG,
```

and X represents a stop codon, that is TAA, TGA or TAG.

**3.** An expression vector comprising a cDNA sequence coding for a compound functioning as porcine pancreatic elastase of the formula:

```
Y-Val Val Gly Gly Thr Glu Ala Gln Arg Asn
    Ser Trp Pro Ser Gln Ile Ser Leu Gln Tyr
    Arg Ser Gly Ser Ser Trp Ala His Thr Cys
    Gly Gly Thr Leu Ile Arg Gln Asn Trp Val
    Met Thr Ala Ala His Cys Val Asp Arg Glu
    Leu Thr Phe Arg Val Val Val Gly Glu His
    Asn Leu Asn Gln Asn Asp Gly Thr Glu Gln
    Tyr Val Gly Val Gln Lys Ile Val Val His
    Pro Tyr Trp Asn Thr Asp Asp Val Ala Ala
    Gly Tyr Asp Ile Ala Leu Leu Arg Leu Ala
    Gln Ser Val Thr Leu Asn Ser Tyr Val Gln
    Leu Gly Val Leu Pro Arg Ala Gly Thr Ile
    Leu Ala Asn Asn Ser Pro Cys Tyr Ile Thr
    Gly Trp Gly Leu Thr Arg Thr Asn Gly Gln
    Leu Ala Gln Thr Leu Gln Gln Ala Tyr Leu
    Pro Thr Val Asp Tyr Ala Ile Cys Ser Ser
    Ser Ser Tyr Trp Gly Ser Thr Val Lys Asn
    Ser Met Val Cys Ala Gly Gly Asp Gly Val
    Arg Ser Gly Cys Gln Gly Asp Ser Gly Gly
    Pro Leu His Cys Leu Val Asn Gly Gln Tyr
    Ala Val His Gly Val Thr Ser Phe Val Ser
    Arg Leu Gly Cys Asn Val Thr Arg Lys Pro
    Thr Val Phe Thr Arg Val Ser Ala Thr Ile
    Ser Trp Ile Asn Asn Val Ile Ala Ser Asn
```

where the group Y is

```
            Thr Gln Asp Phe Pro Glu Thr Asn Ala
Arg, or Met Leu Arg Leu Leu Val Val Ala Ser Leu Val Leu
    Tyr Gly His Ser Thr Gln Asp Phe Pro Glu Thr Asn Ala Arg;
```

and capable of expressing said compound under conditions suitable therefor.

4. A vector according to claim 1 which is pPEX777 as shown in Fig. 5.

5. A host cell transformed with a vector in accordance with any of claims 1 to 4.

6. A transformed host according to claim 5 which is capable of expressing a compound functioning as porcine pancreatic elastase.

7. A process for producing a porcine pancreatic elastase, which comprises culturing a transformed host of claim 6 under conditions resulting in expression of the elastase DNA sequence; and isolating the elastase.

17

**Patentansprüche**

1. Expressionsvektor, umfassend eine cDNA-Sequenz, welche die folgende Basensequenz einschließt und für Schweinepankreas-Elastase codiert:

```
(5')  GTA GTT GGA GGG ACC GAG GCT CAG AGG AAT
      TCT TGG CCA TCT CAG ATT TCC CTC CAG TAC
      CGG TCT GGA AGT TCG TGG GCT CAC ACC TGT
      GGA GGG ACC CTC ATC AGG CAG AAC TGG GTG
      ATG ACA GCC GCT CAC TGC GTG GAC AGA GAG
      TTG ACC TTC CGT GTG GTG GTT GGA GAG CAC
      AAC CTG AAC CAG AAC GAT GGC ACC GAG CAG
      TAC GTG GGG GTG CAG AAG ATC GTG GTG CAT
      CCC TAC TGG AAC ACC GAC GAC GTG GCT GCA
      GGC TAT GAC ATC GCC CTG CTG CGC CTG GCC
      CAG AGT GTA ACC CTC AAC AGC TAC GTC CAG
      CTG GGT GTT CTG CCA AGG GCG GGG ACC ATC
      CTG GCT AAC AAC AGT CCC TGC TAC ATC ACA
      GGC TGG GGC CTG ACC AGG ACC AAT GGG CAG
      CTG GCC CAG ACC CTG CAG CAG GCT TAC CTG
      CCC ACC GTG GAC TAC GCC ATC TGC TCC AGC
      TCC TCG TAC TGG GGC TCC ACC GTG AAG AAC
      AGC ATG GTG TGC GCC GGA GGG GAC GGA GTT
      CGC TCT GGA TGT CAG GGT GAC TCT GGG GGC
      CCC CTT CAT TGC TTG GTG AAT GGT CAG TAT
      GCT GTC CAC GGT GTA ACC AGC TTC GTG TCC
      CGC CTG GGC TGT AAT GTC ACC AGG AAG CCC
      ACA GTC TTC ACC AGG GTC TCT GCT TAC ATC
      TCT TGG ATA AAT AAC GTC ATT GCC AGC AAC  (3')
```

wobei der Expressionsvektor unter dafür geeigneten Bedingungen Schweinepankreas-Elastase exprimieren kann.

2. Expressionsvektor nach Anspruch 1, worin die cDNA aus einer DNA-Sequenz der Formel

```
(5')  Z-GTA GTT GGA GGG ACC GAG GCT CAG AGG AAT
      TCT TGG CCA TCT CAG ATT TCC CTC CAG TAC
      CGC TCT GGA AGT TCG TGG GCT CAC ACC TGT
      GGA GGG ACC CTC ATC AGG CAG AAC TGG GTG
      ATG ACA GCC GCT CAC TGC GTG GAC AGA GAG
      TTG ACC TTC CGT GTG GTG GTT GGA GAG CAC
      AAC CTG AAC CAG AAC GAT GGC ACC GAG CAG
      TAC GTG GGG GGG CAG AAG ATC GTG GTG CAT
      CCC TAC TGG AAC ACC GAC GAC GTG GCT GCA
      GGC TAT GAC ATC GCC CTG CTG CGC CTG GCC
      CAG AGT GTA ACC CTC AAC AGC TAC GTC CAG
      CTG GGT GTT CTG CCA AGG GCG GGG ACC ATC
      CTG GCT AAC AAC AGT CCC TGC TAC ATC ACA
      GGC TGG GGC CTG ACC AGG ACC AAT GGG CAG
      CTG GCC CAG ACC CTG CAG CAG GCT TAC CTG
      CCC ACC GTG GAC TAC GCC ATC TGC TCC AGC
      TCC TCG TAC TGG GGC TCC ACC GTG AAG AAC
      AGC ATG GTG TGC GCC GGA GGG GAC GGA GTT
      CGC TCT GGA TGT CAG GGT GAC TCT GGG GGC
      CCC CTT CAT TGC TTG GTG AAT GGT CAG TAT
      GCT GTC CAC GGT GTA ACC AGC TTC GTG TCC
      CGC CTG GGC TGT AAT GTC ACC AGG AAG CCC
      ACA GTC TTC ACC AGG GTC TCT GCT TAC ATC
      TCT TGG ATA AAT AAC GTC ATT GCC AGC AAC-X (3')
```

besteht, worin Z

```
          ACC CAG GAC TTT CCA GAA ACC AAC GCC
 CGG, oder ATG CTG CGC TTG CTG GTG GTG GCC TCC CTG GTC CTT
 TAT GGA CAC AGC ACC CAG GAC TTT CCA GAA ACC AAC GCC CGG,
```

darstellt und X ein Stopcodon, welches TAA, TGA oder TAG ist, darstellt.

3. Expressionsvektor umfassend eine cDNA-Sequenz, die für eine als Schweinepankreas-Elastase wirkende Verbindung der Formel codiert:

```
Y - Val Val Gly Gly Thr Glu Ala Gln Arg Asn
    Ser Trp Pro Ser Gln Ile Ser Leu Gln Tyr
    Arg Ser Gly Ser Ser Trp Ala His Thr Cys
    Gly Gly Thr Leu Ile Arg Gln Asn Trp Val
    Met Thr Ala Ala His Cys Val Asp Arg Glu
    Leu Thr Phe Arg Val Val Val Gly Glu His
    Asn Leu Asn Gln Asn Asp Gly Thr Glu Gln
    Tyr Val Gly Val Gln Lys Ile Val Val His
    Pro Tyr Trp Asn Thr Asp Asp Val Ala Ala
    Gly Tyr Asp Ile Ala Leu Leu Arg Leu Ala
    Gln Ser Val Thr Leu Asn Ser Tyr Val Gln
    Leu Gly Val Leu Pro Arg Ala Gly Thr Ile
    Leu Ala Asn Asn Ser Pro Cys Tyr Ile Thr
    Gly Trp Gly Leu Thr Arg Thr Asn Gly Gln
    Leu Ala Gln Thr Leu Gln Gln Ala Tyr Leu
    Pro Thr Val Asp Tyr Ala Ile Cys Ser Ser
    Ser Ser Tyr Trp Gly Ser Thr Val Lys Asn
    Ser Met Val Cys Ala Gly Gly Asp Gly Val
    Arg Ser Gly Cys Gln Gly Asp Ser Gly Gly
    Pro Leu His Cys Leu Val Asn Gly Gln Tyr
    Ala Val His Gly Val Thr Ser Phe Val Ser
    Arg Leu Gly Cys Asn Val Thr Arg Lys Pro
    Thr Val Phe Thr Arg Val Ser Ala Thr Ile
    Ser Trp Ile Asn Asn Val Ile Ala Ser Asn
```

wobei der Rest Y

```
        Thr Gln Asp Phe Pro Glu Thr Asn Ala
 Arg, oder Met Leu Arg Leu Leu Val Val Ala Ser Leu Val Leu
 Tyr Gly His Ser Thr Gln Asp Phe Pro Glu Thr Asn Ala Arg
```

ist; und welcher das Molekül unter dafür geeigneten Bedingungen exprimieren kann.

4. Vektor nach Anspruch 1, welcher pPEX777 gemäß Fig. 5 ist.

5. Wirtszelle, die mit einem Vektor gemäß einem der Ansprüche 1 bis 4 transformiert ist.

6. Transformierter Wirt nach Anspruch 5, der eine Verbindung, die als Schweinepankreas-Elastase wirkt, exprimieren kann.

7. Verfahren zur Herstellung von Schweinepankreas-Elastase, welches das Züchten eines transformierten Wirts nach Anspruch 6 unter Bedingungen, welche zur Expression der Elastase-DNA-Sequenz führen, und Isolieren der Elastase umfaßt.

**Revendications**

1. Vecteur d'expression comprenant une séquence d'ADNc contenant la séquence de bases suivante, et codant pour l'élastase pancréatique porcine:

```
(5')  GTA GTT GGA GGG ACC GAG GCT CAG AGG AAT
      TCT TGG CCA TCT CAG ATT TCC CTC CAG TAC
      CGG TCT GGA AGT TCG TGG GCT CAC ACC TGT
      GGA GGG ACC CTC ATC AGG CAG AAC TGG GTG
      ATG ACA GCC GCT CAC TGC GTG GAC AGA GAG
      TTG ACC TTC CGT GTG GTG GTT GGA GAG CAC
      AAC CTG AAC CAG AAC GAT GGC ACC GAG CAG
      TAC GTG GGG GTG CAG AAG ATC GTG GTG CAT
      CCC TAC TGG AAC ACC GAC GAC GTG GCT GCA
      GGC TAT GAC ATC GCC CTG CTG CGC CTG GCC
      CAG AGT GTA ACC CTC AAC AGC TAC GTC CAG
      CTG GGT GTT CTG CCA AGG GCG GGG ACC ATC
      CTG GCT AAC AAC AGT CCC TGC TAC ATC ACA
      GGC TGG GGC CTG ACC AGG ACC AAT GGG CAG
      CTG GCC CAG ACC CTG CAG CAG GCT TAC CTG
      CCC ACC GTG GAC TAC GCC ATC TGC TCC AGC
      TCC TCG TAC TGG GGC TCC ACC GTG AAG AAC
      AGC ATG GTG TGC GCC GGA GGG GAC GGA GTT
      CGC TCT GGA TGT CAG GGT GAC TCT GGG GGC
      CCC CTT CAT TGC TTG GTG AAT GGT CAG TAT
      GCT GTC CAC GGT GTA ACC AGC TTC GTG TCC
      CGC CTG GGC TGT AAT GTC ACC AGG AAG CCC
      ACA GTC TTC ACC AGG GTC TCT GCT TAC ATC
      TCT TGG ATA AAT AAC GTC ATT GCC AGC AAC (3');
```

le vecteur d'expression étant capable d'exprimer l'élastase pancréatique porcine, dans des conditions appropriées à cette fin.

2. Vecteur d'expression selon la revendication 1, dans lequel l'ADNc consiste en une séquence d'ADN de formule

```
(5')  Z-GTA GTT GGA GGG ACC GAG GCT CAG AGG AAT
      TCT TGG CCA TCT CAG ATT TCC CTC CAG TAC
      CGC TCT GGA AGT TCG TGG GCT CAC ACC TGT
      GGA GGG ACC CTC ATC AGG CAG AAC TGG GTG
      ATG ACA GCC GCT CAC TGC GTG GAC AGA GAG
      TTG ACC TTC CGT GTG GTG GTT GGA GAG CAC
      AAC CTG AAC CAG AAC GAT GGC ACC GAG CAG
      TAC GTG GGG GGG CAG AAG ATC GTG GTG CAT
      CCC TAC TGG AAC ACC GAC GAC GTG GCT GCA
      GGC TAT GAC ATC GCC CTG CTG CGC CTG GCC
      CAG AGT GTA ACC CTC AAC AGC TAC GTC CAG
      CTG GGT GTT CTG CCA AGG GCG GGG ACC ATC
      CTG GCT AAC AAC AGT CCC TGC TAC ATC ACA
      GGC TGG GGC CTG ACC AGG ACC AAT GGG CAG
      CTG GCC CAG ACC CTG CAG CAG GCT TAC CTG
      CCC ACC GTG GAC TAC GCC ATC TGC TCC AGC
      TCC TCG TAC TGG GGC TCC ACC GTG AAG AAC
      AGC ATG GTG TGC GCC GGA GGG GAC GGA GTT
      CGC TCT GGA TGT CAG GGT GAC TCT GGG GGC
      CCC CTT CAT TGC TTG GTG AAT GGT CAG TAT
      GCT GTC CAC GGT GTA ACC AGC TTC GTG TCC
      CGC CTG GGC TGT AAT GTC ACC AGG AAG CCC
      ACA GTC TTC ACC AGG GTC TCT GCT TAC ATC
      TCT TGG ATA AAT AAC GTC ATT GCC AGC AAC-X (3')
```

dans laquelle Z représente

$$ACC\ CAG\ GAC\ TTT\ CCA\ GAA\ ACC\ AAC$$
$$GCC\ CGG\ \text{ou}\ ATG\ CTG\ CGC\ TTG\ CTG\ GTG\ GTG\ GCC\ TCC\ CTG\ GTC\ CTT$$
$$TAT\ GGA\ CAC\ AGC\ ACC\ CAG\ GAC\ TTT\ CCA\ GAA\ ACC\ AAC\ GCC\ CGG,$$

et X représente un codon d'arrêt, à savoir TAA, TGA ou TAG.

   **3.** Vecteur d'expression comprenant une séquence d'ADNc codant pour un composé, fonctionnant en tant qu'élastase pancréatique porcine, de formule:

```
Y-Val Val Gly Gly Thr Glu Ala Gln Arg Asn
     Ser Trp Pro Ser Gln Ile Ser Leu Gln Tyr
     Arg Ser Gly Ser Ser Trp Ala His Thr Cys
     Gly Gly Thr Leu Ile Arg Gln Asn Trp Val
     Met Thr Ala Ala His Cys Val Asp Arg Glu
     Leu Thr Phe Arg Val Val Val Gly Glu His
     Asn Leu Asn Gln Asn Asp Gly Thr Glu Gln
     Tyr Val Gly Val Gln Lys Ile Val Val His
     Pro Tyr Trp Asn Thr Asp Asp Val Ala Ala
     Gly Tyr Asp Ile Ala Leu Leu Arg Leu Ala
     Gln Ser Val Thr Leu Asn Ser Tyr Val Gln
     Leu Gly Val Leu Pro Arg Ala Gly Thr Ile
     Leu Ala Asn Asn Ser Pro Cys Tyr Ile Thr
     Gly Trp Gly Leu Thr Arg Thr Asn Gly Gln
     Leu Ala Gln Thr Leu Gln Gln Ala Tyr Leu
     Pro Thr Val Asp Tyr Ala Ile Cys Ser Ser
     Ser Ser Tyr Trp Gly Ser Thr Val Lys Asn
     Ser Met Val Cys Ala Gly Gly Asp Gly Val
     Arg Ser Gly Cys Gln Gly Asp Ser Gly Gly
     Pro Leu His Cys Leu Val Asn Gly Gln Tyr
     Ala Val His Gly Val Thr Ser Phe Val Ser
     Arg Leu Gly Cys Asn Val Thr Arg Lys Pro
     Thr Val Phe Thr Arg Val Ser Ala Thr Ile
     Ser Trp Ile Asn Asn Val Ile Ala Ser Asn
```

dans laquelle le groupe Y est

```
                    Thr Gln Asp Phe Pro Glu Thr
Asn Ala Arg, ou Met Leu Arg Leu Leu Val Val Ala Ser Leu Val
Leu Tyr Gly His Ser Thr Gln Asp Phe Pro Glu Thr Asn Ala Arg,
```

et capable d'exprimer ledit composé dans des conditions appropriées à cette fin.

4. Vecteur selon la revendication 1, qui est pPEX777, tel que représenté sur la figure 5.

5. Cellule hôte transformée par un vecteur selon l'une quelconque des revendications 1 à 4.

6. Hôte transformé selon la revendication 5, qui est capable d'exprimer un composé fonctionnant en tant qu'élastase pancréatique porcine.

7. Procédé pour la production d'une élastase pancréatique porcine, comprenant la culture d'un hôte transformé de la revendication 6, dans des conditions conduisant à l'expression de la séquence d'ADN de l'élastase et l'isolement de l'élastase.

(5')-AGTGGTCTTCTCAGCTAGCAAC ATG CTG CGC TTG CTG GTG
                            Met Leu Arg Leu Leu Val
                                                 -01
GTG GCC TCC CTG GTC CTT TAT GGA CAC AGC ACC CAG GAC TTT CCA GAA ACC AAC GCC CGG
Val Ala Ser Leu Val Leu Tyr Gly His Ser Thr Gln Asp Phe Pro Glu Thr Asn Ala Arg
                                                                             20
GTA GTT GGA GGG ACC GAG GCT CAG AGG AAT TCT TGG CCA TCT CAG ATT TCC CTC CAG TAC
Val Val Gly Gly Thr Glu Ala Gln Arg Asn Ser Trp Pro Ser Gln Ile Ser Leu Gln Tyr
                                                                             40
CGG TCT GGA AGT TCG TGG GCT CAC ACC TGT GGA GGG ACC CTC ATC AGG CAG AAC TGG GTG
Arg Ser Gly Ser Ser Trp Ala His Thr Cys Gly Gly Thr Leu Ile Arg Gln Asn Trp Val
                                                                             60
ATG ACA GCC GCT CAC TGC GTG GAC AGA GAG TTG ACC TTC CGT GTG GTG GTT GGA GAG CAC
Met Thr Ala Ala His Cys Val Asp Arg Glu Leu Thr Phe Arg Val Val Val Gly Glu His
                                                                             80
AAC CTG AAC CAG AAC GAT GGC ACC GAG CAG TAC GTG GGG GTG CAG AAG ATC GTG GTG CAT
Asn Leu Asn Gln Asn Asp Gly Thr Glu Gln Tyr Val Gly Val Gln Lys Ile Val Val His
                                                                            100
CCC TAC TGG AAC ACC GAC GAC GTG GCT GCA GGC TAT GAC ATC GCC CTG CTG CGC CTG GCC
Pro Tyr Trp Asn Thr Asp Asp Val Ala Ala Gly Tyr Asp Ile Ala Leu Leu Arg Leu Ala
                                                                            120
CAG AGT GTA ACC CTC AAC AGC TAC GTC CAG CTG GGT GTT CTG CCA AGG GCG GGG ACC ATC
Gln Ser Val Thr Leu Asn Ser Tyr Val Gln Leu Gly Val Leu Pro Arg Ala Gly Thr Ile

......Continued

FIG.I .

EP 0 157 604 B1

```
                                                                        140
CTG GCT AAC AAC AGT CCC TGC TAC ATC ACA GGC TGG GGC CTG ACC AGG ACC AAT GGG CAG
Leu Ala Asn Asn Ser Pro Cys Tyr Ile Thr Gly Trp Gly Leu Thr Arg Thr Asn Gly Gln
                                                                        160
CTG GCC CAG ACC CTG CAG CAG GCT TAC CTG CCC ACC GTG GAC TAC GCC ATC TGC TCC AGC
Leu Ala Gln Thr Leu Gln Gln Ala Tyr Leu Pro Thr Val Asp Tyr Ala Ile Cys Ser Ser
                                                                        180
TCC TCG TAC TGG GGC TCC ACC GTG AAG AAC AGC ATG GTG TGC GCC GGA GGG GAC GGA GTT
Ser Ser Tyr Trp Gly Ser Thr Val Lys Asn Ser Met Val Cys Ala Gly Gly Asp Gly Val
                                                                        200
CGC TCT GGA TGT CAG GGT GAC TCT GGG GGC CCC CTT CAT TGC TTG GTG AAT GGT CAG TAT
Arg Ser Gly Cys Gln Gly Asp Ser Gly Gly Pro Leu His Cys Leu Val Asn Gly Gln Tyr
                                                                        220
GCT GTC CAC GGT GTA ACC AGC TTC GTG TCC CGC CTG GGC TGT AAT GTC ACC AGG AAG CCC
Ala Val His Gly Val Thr Ser Phe Val Ser Arg Leu Gly Cys Asn Val Thr Arg Lys Pro
                                                                        240
ACA GTC TTC ACC AGG GTC TCT GCT TAC ATC TCT TGG ATA AAT AAC GTC ATT GCC AGC AAC
Thr Val Phe Thr Arg Val Ser Ala Tyr Ile Ser Trp Ile Asn Asn Val Ile Ala Ser Asn

TGA ACATTTTCCGGAGTCCAACGGTCTTCCGAGGTGACTCTTAGATGCTCAGCAGGACTTGAGGCCACCAAAGAAATT

ACATTCTAAGAGACTATTGAGTCAGATACAGAAAGCAATAAACCGAATATACATATA(A)n-(3')
```

# FIG.I .

FIG. 2 .

Pst I   Xma I   Eco RI   Sau 3A   Pst I   Pst I   Hinf I   Hinf I   Hinf I   Hinf I   Pst I

0   500   1000

(base pairs)

EP 0 157 604 B1

```
          activation peptide                          mature elastase
 _____    _____

Thr Gln Asp Phe Pro Glu Thr Asn Ala Arg Val Val Gly Gly Thr Glu Ala
ACC CAG GAC TTT CCA GAA ACC AAC GCC CGG GTA GTT GGA GGG ACC GAG GCT
                                    Xma I
```

## FIG. 3.

```
                                β-galactosidase
                    _____

                    Met Thr Met Ile Thr Asn Ser Arg Gly Ser Val Asp
ACA CAG GAA ACA GCT ATG ACC ATG ATT ACG AAT TCC CGG GGA TCC GTC GAC
                                            Xma I
```

## FIG. 4.

```
          β-galactosidase                        mature elastase
 _____    _____

          Met Thr Met Ile Thr Asn Ser Arg Val Val Gly Gly Thr Glu Ala
ACA GCT ATG ACC ATG ATT ACG AAT TCC CGG GTA GTT GGA GGG ACC GAG GCT
                                    Xma I
```

## FIG. 6.

FIG. 5.